# DEMANDE DE BREVET EUROPEEN

(11) **EP 1 321 451 A1**
(43) Date de publication de la demande: **25.06.2003**
(21) Numéro de dépôt: 02293068.9
(22) Date de dépôt: 12.12.2002
(51) Int. Cl.: C07C 68/02, C07C 329/02, C07C 69/96

(54) **Procédé de préparation de fluoroformiates**

(30) Priorité: 20.12.2001 FR 0116514
(71) Demandeur: ISOCHEM, 75004 Paris (FR)
(72) Inventeur: Alarcon-Lorca, Alphonse, 91450 Soisy sur Seine (FR); Malfroot, Thierry, 91250 Saintry sur Seine (FR); Senet, Jean-Pierre, Herbeauvilliers, 77760 Buthiers (FR)
(74) Mandataire: Waligorski, Carol

(57) **Abrégé**

L'invention concerne un procédé de préparation des fluoroformiates de formule 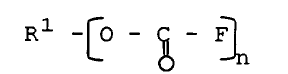 dans laquelle
R¹ représente un radical aliphatique primaire ou secondaire, saturé ou non, substitué ou non, un radical cycloaliphatique secondaire, saturé ou non, substitué ou non, ou un radical aromatique ou hétéroaromatique substitué ou non, et
n est égal à 1, 2 ou 3,
caractérisé en ce que l'on fait réagir le chloroformiate correspondant avec un fluorure de métal alcalin dans le carbonate d'éthylène, à une température à laquelle le carbonate d'éthylène est liquide.

Ce procédé permet d'obtenir les fluoroformiates très rapidement, avec d'excellents rendements et une grande pureté.

## Description

La présente invention concerne un procédé amélioré de préparation de fluoroformiates. Elle concerne en particulier le procédé amélioré de préparation de fluoroformiates par échange du chlore par le fluor dans des chloroformiates.

Les fluoroformiates sont des composés connus. Ils sont très utiles comme intermédiaires de synthèse de composés organiques employés en agriculture ou en pharmacie. Ils permettent notamment l'introduction des radicaux oxycarbonylés dans les molécules et remplacent avantageusement les chloroformiates qui sont souvent instables dans les conditions réactionnelles utilisées.

Un des procédés de préparation des fluoroformiates consiste à faire réagir un fluorohalogénure de carbonyle avec un alcool ou un phénol. Mais ce procédé présente plusieurs inconvénients. Les halogénures de fluorocarbonyle sont très difficiles à préparer et donc très peu courants et très chers. Comme ils ne peuvent pas être stockés, il faut les fabriquer juste avant leur utilisation. Leur manipulation est également difficile et dangereuse. Une installation spéciale est nécessaire et des températures très basses sont utilisées. De plus, le fluorure de sodium employé doit avoir une granulométrie particulière. Le procédé est par conséquent peu économique.

Une autre préparation décrite par J. Cuomo et R.A. Olofson (J. Org. Chem., vol. 44, No. 6, 1979) s'effectue à partir des chloroformiates par échange du chlore par le fluor. La réaction est effectuée au moyen du fluorure de potassium en présence d'un catalyseur pour l'activer tel que l'éther-couronne 18-C-6 qui complexe le potassium. Mais d'une part les éthers-couronnes ne sont pas des composés courants et ils sont par conséquent vendus à des prix très élevés, d'autre part les durées de réaction indiquées sont très élevées, de 45 heures à 73 heures pour les fluoroformiates aliphatiques, de 144 heures pour les fluoroformiates aromatiques comme le fluoroformiate de phényle.

Des améliorations à ce procédé ont alors été proposées par J. Ichihara et al (J. Chem. Soc., Chem. Commun., 1986, pp 793-4). Elles consistent à utiliser un mélange calciné de fluorure de potassium et de fluorure de calcium, dans un solvant tel que l'acétonitrile. La durée de réaction pour préparer le fluoroformiate d'éthyle est encore très élevée, de 24 heures. Au préalable, les mélanges calcinés de fluorures doivent être soigneusement préparés selon une procédure particulière et à température élevée.

Il existait par conséquent un besoin d'obtenir les fluoroformiates à partir des chloroformiates correspondants dans de meilleures conditions que celles des procédés de l'art antérieur, et notamment avec des temps de réaction beaucoup plus courts.

Selon le procédé de l'invention, on prépare les fluoroformiates de formule 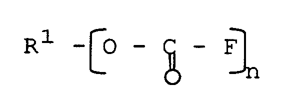 dans laquelle
R¹ représente un radical aliphatique primaire ou secondaire, saturé ou non, substitué ou non, un radical cycloaliphatique secondaire saturé ou non, substitué ou non, ou un radical aromatique ou hétéroaromatique substitué ou non, et
n est égal à 1, 2 ou 3,
par réaction du chloroformiate correspondant avec un fluorure de métal alcalin dans le carbonate d'éthylène, à une température à laquelle le carbonate d'éthylène est liquide.

En mettant en oeuvre le procédé dans ces conditions les fluoroformiates sont obtenus très rapidement. La durée de réaction est généralement de 1 à 2 heures aussi bien pour les fluoroformiates aliphatiques que pour les fluoroformiates aromatiques. Leurs rendements et leurs puretés sont excellents et souvent nettement améliorés par rapport à ceux de l'art antérieur.

Les chloroformiates qui sont utilisés comme composés de départ sont disponibles dans le commerce ou sont préparés selon des méthodes connues en soi.

De façon préférée, le procédé selon l'invention est réalisé en ajoutant le chloroformiate dans le mélange du fluorure alcalin et du carbonate d'éthylène. Pour de meilleurs résultats, cet ajout est effectué de façon progressive.

Le procédé selon l'invention est bien adapté pour préparer les fluoroformiates dont les chloroformiates de départ sont stables dans les milieux très polaires et sont liquides à la température ordinaire.

Il convient particulièrement bien également lorsque les fluoroformiates que l'on souhaite obtenir sont liquides à la température ordinaire et lorsqu'ils ont un point d'ébullition nettement inférieur à celui du carbonate d'éthylène.

Les chloroformiates utiles sont notamment les chloroformiates aliphatiques primaires et secondaires, les chloroformiates cycloaliphatiques non tertiaires, les chloroformiates aromatiques ou hétéroaromatiques ne portant pas de groupes très électroattracteurs sur le noyau cyclique.

Le radical R¹ peut notamment représenter un radical aliphatique primaire ou secondaire et en particulier un radical en C₂ à C₁₀, substitué ou non. De préférence, il contient de 2 à 8 atomes de carbone.

Des doubles ou triples liaisons carbone-carbone peuvent être présentes, à condition que la double ou la triple liaison ne soit par sur le carbone en position 2, c'est-à-dire que ce ne soit pas par exemple un radical de type allylique ou propargylique.

La chaîne aliphatique peut être linéaire ou ramifiée. Elle peut contenir des hétéroatomes tels que des atomes d'oxygène ou de soufre. Les substituants peuvent être choisis notamment parmi les radicaux cycloaliphatiques, halogénés ou non, ou aromatiques, halogénés ou non, à condition que ces derniers ne soient pas fixés sur le carbone portant le groupe formiate, les atomes d'halogène à condition qu'ils ne soient pas fixés sur le carbone portant le groupe formiate, les groupes alcoxy ou aryloxy halogénés ou non.

Comme exemples de radicaux, on peut citer les radicaux éthyle, n- ou i-propyle, vinyle, isopropényle, n- ou i-butyle, le radical dérivé du diéthylèneglycol.

Lorsque R¹ représente un radical cycloaliphatique, celui-ci peut être polycyclique. De préférence, le cycle attaché au groupe formiate contient de 4 à 7 atomes de carbone.

Il peut être insaturé avec les mêmes restrictions que pour le radical aliphatique.

Les substituants du ou des cycles aliphatiques peuvent être choisis parmi les atomes d'halogène, les radicaux aliphatiques, cycloaliphatiques, aromatiques, alcoxy ou aryloxy, halogénés ou non. Comme radical cycloaliphatique, on peut citer, en particulier, le radical cyclopentyle, cyclohexyle, adamantyle ou cholestéryle.

R¹ peut également représenter un radical aromatique ou hétéroaromatique mono- ou polycylique. Le ou les cycles peuvent porter des substituants notamment choisis parmi les atomes d'halogène, les radicaux aliphatiques, cycloaliphatiques, aromatiques, alkoxy ou aryloxy, halogénés ou non et similaires. Lorsque les substituants sont très électroattracteurs, ils conviennent en général moins.

Les radicaux hétéroaromatiques sont en particulier ceux dont l'hétérocycle a 5 ou 6 sommets. Les hétéroatomes du ou des cycles sont choisis parmi les atomes d'azote, de soufre ou d'oxygène, de préférence parmi les atomes d'oxygène ou de soufre.

Comme radicaux aromatiques, on peut en particulier citer les radicaux phényle ou naphtyle, substitués ou non.

Comme radicaux hétéroaromatiques, on peut en particulier citer les radicaux furyle, pyrrolyle, thiényle, pyridyle et pyrimidinyle, substitués ou non.

Comme atomes d'halogène, on choisit de préférence les atomes de chlore, de fluor ou de brome.

Sur les radicaux R¹ peuvent être attachés un, deux ou trois groupes formiates.

Comme composé bis-fluoroformiate, on peut notamment citer le bis-fluoroformiate de diéthylèneglycol.

L'échange chlore-fluor est effectué au moyen des fluorures alcalins. Ceux-ci se trouvent dans le commerce. Ils sont généralement utilisés sous forme de poudre afin d'accélérer leur dissolution dans le carbonate d'éthylène.

Le métal alcalin est de préférence le sodium ou le potassium et plus particulièrement le potassium.

La quantité de fluorure alcalin par rapport au chloroformiate n'est pas critique. Selon la stoechiométrie de la réaction, il est souhaitable que soit présent dans la réaction au moins un atome de fluor par atome de chlore à remplacer. De préférence, on utilise de 1,2 à 2 équivalents molaires de fluorure alcalin par rapport au chloroformiate.

Pour obtenir les avantages cités précédemment, il est nécessaire que la réaction ait lieu dans le carbonate d'éthylène.

Le carbonate d'éthylène est un composé solide à la température ordinaire. Compte tenu de sa structure, on pouvait craindre qu'il se décarboxyle facilement ou qu'il réagisse avec les composés présents dans le milieu.

Or on a maintenant trouvé que son utilisation améliorait très nettement la réaction entre le chloroformiate et le fluorure alcalin, lorsqu'on l'utilisait à l'état liquide comme solvant, c'est-à-dire lorsque la réaction est effectuée à une température égale ou supérieure à son point de fusion qui est en particulier de 37°-39° C sous la pression atmosphérique normale.

Il n'est de plus pas nécessaire pour accélérer la réaction d'utiliser des températures élevées, par exemple supérieures à 100° C, notamment pour préparer les fluoroformiates aromatiques. La réaction peut être effectuée à une température comprise entre la température de fusion du carbonate d'éthylène et environ 60° C. De préférence, on choisit une température comprise entre 40° et 50° C ou égale à ces valeurs.

La quantité de carbonate d'éthylène n'est pas un facteur déterminant. Elle doit être suffisante pour permettre une bonne agitation du milieu. Une quantité excessive entraîne une baisse de productivité et une décomposition très importante des chloroformiates. Généralement on utilise de 2,5 à 5 équivalents de carbonate d'éthylène.

Pour la mise en oeuvre du procédé, on mélange généralement tout d'abord le fluorure alcalin et le carbonate d'éthylène. On ajoute ensuite le chloroformiate dans le mélange ainsi formé et agité. De préférence, l'addition se fait progressivement dans le mélange.

La réaction peut être effectuée à la pression atmosphérique. De préférence, elle se déroule dans des conditions anhydres.

La durée de la réaction est considérablement plus faible que dans les procédés antérieurs.

Ainsi pour les fluoroformiates aliphatiques et cycloaliphatiques, la durée de la réaction était selon la méthode utilisée par J. Cuomo. et al, de 45 h à 73 h. Grâce à l'invention, elle est d'environ 1 à 2 heures. De même, le fluoroformiate de cyclohexyle était obtenu auparavant en 4 heures à une température de 70° C. Il l'est maintenant en à peine une heure à une température de 45° C.

Les fluoroformiates obtenus peuvent être récupérés par des méthodes usuelles. Généralement, on évapore les composés volatils, sous pression réduite, en faisant varier la température et la pression dans le réacteur. On recueille les composés volatils dans un récepteur maintenu à basse température, telle que de l'ordre de -50° à -70° C.

Si nécessaire, on effectue une distillation pour purifier le fluoroformiate.

Les rendements en fluoroformiates sont excellents et en général améliorés par rapport à ceux de la technique antérieure.

Le fluoroformiate de phényle par exemple était récupéré en 144 h, avec un rendement de 80 % seulement, il l'est maintenant en environ 2 heures avec un rendement nettement supérieur, de 93,5 %.

La pureté des fluoroformiates obtenue est excellente et une distillation n'est pas toujours nécessaire.

Les exemples qui suivent illustrent l'invention sans toutefois la limiter.

### EXEMPLE 1 : PREPARATION DU FLUOROFORMIATE D'ISOPROPYLE

Dans un réacteur en verre de 20 1 équipé, on introduit 8938 g (106,4 mol) de carbonate d'éthylène et on les sèche sous agitation pendant 5 heures à une température de 90°C, sous une pression de 80 mm Hg. On ajoute ensuite 3306 g (57 mol) de fluorure de potassium en poudre préalablement séché à 100°C sous 1 mm Hg pendant 5 heures. On refroidit le réacteur et on introduit sous azote et à la pression atmosphérique, en 2 heures, 4655 g (38 mol) de chloroformiate d'isopropyle dans le milieu réactionnel maintenu entre 40° et 45°C. L'avancement de la réaction est contrôlé par chromatographie en phase gazeuse (CPG).

On constate que la réaction est terminée à la fin de l'introduction du chloroformiate. On récupère le fluoroformiate formé, dans un récepteur maintenu aux environs de -60°C, par évaporation sous vide, en faisant varier la température dans le réacteur de 50° à 82°C et la pression de 760 à 35 mm Hg . On le purifie par distillation sous pression réduite et on recueille ainsi 3750 g de fluoroformiate d'isopropyle (rendement 93%). Sa pureté déterminée par CPG est de 99,9%. Le taux de fluor hydrolysable est de 100%.

### EXEMPLE 2 : PREPARATION DE FLUOROFORMIATE DE n-OCTYLE

On opère comme à l'exemple 1, mais en utilisant 100 g de carbonate d'éthylène, 30 g (0,52 mol) de fluorure de potassium et 67,5 g (0,35 mol) de chloroformiate de n-octyle.

Après addition du chloroformiate, on continue à agiter le mélange réactionnel à 45°C, pendant 1,5 h. L'évaporation sous vide est effectuée à une température variant de 50° à 100°C, sous une pression de 760 à 10 mm Hg. Par distillation, sous pression réduite, des composés recueillis, on récupère 54,9 g (rendement 89%) de fluoroformiate de n-octyle (Pt Ebullition, (Eb) : 87°C/14 mm Hg).

### EXEMPLE 3 : PREPARATION DU FLUOROFORMIATE DE NEOPENTYLE

On opère comme à l'exemple 2, mais en utilisant 120 g de carbonate d'éthylène, 45 g (0,77 mol) de fluorure de potassium et 75,3 g (0,5 mol) de chloroformiate de néopentyle.

Après addition du chloroformiate, on continue à agiter le mélange réactionnel pendant 1 heure, à 45°C. L'évaporation sous vide est effectuée comme à l'exemple précédent. Par distillation, sous pression réduite, on récupère 61,7 g (rendement 92%) de fluoroformiate de néopentyle (Eb : 60°C/20 mm Hg).

### EXEMPLE 4 : PREPARATION DU FLUOROFORMIATE DE PHENYLE

On opère comme à l'exemple 2, mais en utilisant 250 g de carbonate d'éthylène, 89 g (1,53 mol) de fluorure de potassium et 156,6 g (1,0 mol) de chloroformiate de phényle.

Après addition du chloroformiate, on continue à agiter le mélange réactionnel, à 45°-50°C, pendant 2 heures. L'évaporation sous vide est effectuée comme précédemment. Par distillation, sous pression réduite, on récupère 131 g (rendement 93,5%) de fluoroformiate de phényle (Eb : 62°-64°C/40 mm Hg).

### EXEMPLE 5 : PREPARATION DU FLUOROFORMIATE DE CYCLOHEXYLE

On opère comme à l'exemple 2, mais en utilisant 250 g de carbonate d'éthylène, 90 g (1,55 mol) de fluorure de potassium et 162,6 g (1,0 mol) de chloroformiate de cyclohexyle.

Après addition du chloroformiate, on continue à agiter le mélange pendant 0,75 h, à 45°C. L'évaporation sous vide est effectuée comme précédemment. Par distillation, sous pression réduite, on récupère 122,7 g (rendement 84%) de fluoroformiate de cyclohexyle (Eb : 55-56°C/22 mm Hg).

## Revendications

1. Procédé de préparation des fluoroformiates de formule 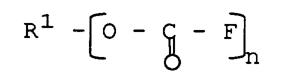 dans laquelle
R¹ représente un radical aliphatique primaire ou secondaire, saturé ou non, substitué ou non, un radical cycloaliphatique secondaire saturé ou non, substitué ou non, ou un radical aromatique ou hétéroaromatique substitué ou non, et
n est égal à 1, 2 ou 3,
**caractérisé en ce que** l'on fait réagir le chloroformiate correspondant avec un fluorure de métal alcalin dans le carbonate d'éthylène, à une température à laquelle le carbonate d'éthylène est liquide.

2. Procédé selon la revendication 1, **caractérisé en ce que** le chloroformiate est introduit dans le mélange du fluorure alcalin et du carbonate d'éthylène.

3. Procédé selon la revendication 2, **caractérisé en ce que** l'introduction du chloroformiate est effectuée de façon progressive.

4. Procédé selon l'une quelconque des revendications précédente, **caractérisé en ce que** le fluorure alcalin est le fluorure de sodium ou de potassium.

5. Procédé selon l'une quelconque des revendications précédentes, **caractérisé en ce que** le fluorure alcalin est sous forme de poudre.

6. Procédé selon l'une quelconque des revendications précédentes, **caractérisé en ce que** la température de la réaction est comprise entre la température de fusion du carbonate d'éthylène et 60°C.
